# EUROPEAN PATENT APPLICATION

(11) **EP 3 744 374 A1**
(43) Date of publication of application: **02.12.2020**
(21) Application number: 19743817.9
(22) Date of filing: 23.01.2019
(51) Int. Cl.: A61M 5/42, A61B 5/15, A61M 37/00

(54) **TIGHT-BINDING DEVICE FOR MICRO-NEEDLE**

(30) Priority: 24.01.2018 JP 2018023525
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2019/002011
(87) International publication number: WO 2019/146618

(57) **Abstract**

Provided is an auxiliary tool that makes skin administration of a micro-needle array on which a large number of micro-needles are provided as a result of a progress in fine processing technology more certain. A skin tight-binding device provided with a tight-binding band, and a hole provided on the tight-binding band, in which, when the tight-binding band is fixed to skin, the hole forms a micro-needle patch administration portion, and the skin tight-binding device further provided with a fixing tool that fixes the tight-binding band to the skin.

## Description

### TECHNICAL FIELD

The present invention relates to a device to be attached to skin when a micro-needle is administered to the skin.

### BACKGROUND ART

Oral administration and transdermal administration are often used as a method of administering a drug into a human body. Injection is a representative transdermal administrating method, but this is an unwelcome method that is cumbersome and painful and might cause infection. In a case of the transdermal administration, the skin stratum corneum serves as a barrier for drug permeation, so that simple application of a drug to a skin surface does not always have sufficient permeability. In contrast, by piercing the stratum corneum with a fine needle, that is, a micro-needle, drug permeation efficiency may be significantly improved as compared with that in a coating method. A large number of micro-needles are accumulated on a substrate to obtain a micro-needle array. An adhesive sheet for adhering the micro-needle array to the skin, a protective release sheet for protecting the adhesive sheet and serving as a support when adhering the micro-needle array to the skin and the like are added to this to obtain an easy-to-use product, which is referred to as a micro-needle patch.

When the micro-needle array is administered to the skin, the skin is generally soft and puncture with the micro-needle is not easy just by merely pressing the same with a finger, so that an applicator is often used as a puncture aid.

In order to administer the micro-needle array to the skin having shock absorption capability, it is necessary to administer the micro-needle array to the skin at a high speed and with an impact. As this method, use of a spring (Patent Documents 1-6), an air pressure (Patent Document 5), a magnetic force (Patent Document 7) and the like has been proposed so far.

The present inventors have developed a simple and easy-to-use spring-type micro-needle patch administration device capable of certainly administering the micro-needle patch to the skin having shock absorption capability (Patent Document 8) .

In contrast, when collecting blood using a hollow single-needle syringe, a tourniquet or other auxiliary tool for tightening around an arm is used. As an example, an auxiliary tool wound around the arm for increasing a blood volume from the skin, a blood collecting auxiliary tool provided with a compression band that compresses the skin surface and a belt that fixes the compression band and including a ring for tensing a skin portion from which the blood is collected on an arm distal side of the compression band is known (Patent Document 9).

### PRIOR ART DOCUMENTS

### PATENT DOCUMENTS

Patent Document 1: JP 2004-510530 A (JP 4198985 B2)
Patent Document 2: JP 2004-510534 A (JP 4104975 B2)
Patent Document 3: JP 2004-510535 A (JP 4659332 B2)
Patent Document 4: JP 2005-533625 A
Patent Document 5: JP 2006-500973 A
Patent Document 6: JP 2007-509706 A (JP 4682144 B2)
Patent Document 7: JP 2011-078711 A
Patent Document 8: JP 2014-042788 A (JP 6091818 B2)
Patent Document 9: JP 2000-237171 A

### SUMMARY OF THE INVENTION

### PROBLEM TO BE SOLVED BY THE INVENTION

In the course of research into micro-needle development, it has been found that the larger the number of micro-needles on the array, the more difficult the piercing of the skin even with the applicator. With an increase in needle density, a phenomenon has occurred that the skin is deformed inward by pressing the skin with all the needles, but a part of the needles is not inserted into the skin. A problem to be solved by the present invention is to provide an auxiliary tool for more certainly administering into the skin the micro-needle array in which a large number of micro-needles are provided due to a progress in fine processing technology.

### MEANS FOR SOLVING THE PROBLEM

In order to solve the above-described problem, the present inventors have exhaustively studied more stable intradermal insertion of the micro-needle to find that the intradermal insertion of the micro-needle is certain when hardness (tension) is given to the skin using the auxiliary tool, thereby achieving the present invention.

The present invention is as follows.
[1] A skin tight-binding device provided with a tight-binding band, and a hole provided on the tight-binding band, in which, when the tight-binding band is fixed to skin, the hole forms a micro-needle patch administration portion.
[2] The skin tight-binding device according to [1], further provided with a fixing tool that fixes the tight-binding band to the skin.
[3] The skin tight-binding device according to [2], in which the tight-binding band and the fixing tool are integrally connected to each other to form the skin tight-binding device.
[4] The skin tight-binding device according to any one of [1] to [3], in which the hole provided on the tight-binding band is formed after the skin tight-binding device is attached to the skin.
[5] The skin tight-binding device according to any one of [1] to [4], in which, in a case of being attached to the skin, a degree of tension of the skin in the hole is 0.9 or smaller as a skin deformation ratio as compared to the degree before the attachment.
[6] The skin tight-binding device according to any one of [1] to [4], in which, in a case of being attached to the skin, a degree of tension of the skin in the hole is 0.9 or smaller as a depression degree ratio as compared to the degree before the attachment.
[7] The skin tight-binding device according to any one of [1] to [6], in which the tight-binding band or the fixing tool is a stretchable cloth at least partially.
[8] The skin tight-binding device according to any one of [2] to [7], in which the fixing tool is a stretchable cloth, and the tight-binding band is a non-stretchable cloth.

### EFFECT OF THE INVENTION

As a device for compressing skin to further ensure an effect, there is a tourniquet used when blood is collected using a syringe. This device is for stopping bleeding and filling the peripheral venous blood vessel with blood. In contrast, a skin tight-binding device of the present invention maintains constant skin hardness (tension) in an administration site of the skin to make intradermal insertion of a micro-needle patch easy and certain by this effect. In a state in which the skin is slack or relaxed, the micro-needle often remains on a skin surface without breaking the skin because the skin is depressed even when the micro-needle is administered. That is, the intradermal insertion of the micro-needle becomes uncertain. On the contrary, when the skin tight-binding device of the present invention is used to give an appropriate tension state to the skin and the micro-needle patch is administered with an applicator, the skin accepts the needle without sagging, and certain intradermal administration of the needle is realized. Note that, the skin tight-binding device of the present invention is supposed to be removed after the micro-needle patch is properly adhered to the skin with a protective adhesive tape or the like after the administration of the micro-needle.

In summary, the skin tight-binding device of the present invention ensures the insertion of a large number of needles into the skin when administering the patch including a micro-needle array on which a large number of micro-needles are provided, and a drug contained in the micro-needles may be efficiently delivered intradermally.

### BRIEF DESCRIPTION OF THE DRAWINGS

FIG. 1(A) is a perspective view illustrating an embodiment of a skin tight-binding device (with a wire ring) of the present invention, and FIG. 1(B) is an enlarged cross-sectional view of a wire ring portion provided around a hole of the skin tight-binding device of the present invention.
FIG. 2 is a perspective view illustrating an embodiment of a skin tight-binding device (without a ring) of the present invention.

### MODE FOR CARRYING OUT THE INVENTION

A skin tight-binding device of the present invention is provided with a tight-binding band and a hole provided on the tight-binding band, in which, when the tight-binding band is fixed to skin, the hole forms a micro-needle patch administration portion. The skin tight-binding device of the present invention may further be provided with a fixing tool that fixes the tight-binding band to the skin. The skin tight-binding device of the present invention may be attached to an administration site of the skin before administration of a micro-needle patch and removed after the administration. That is, the skin tight-binding device of the present invention is an auxiliary tool used when administering the micro-needle patch.

The tight-binding band typically has a rectangular or band shape, but may have any shape and any size depending on a size of the micro-needle patch and the administration site. When used on an upper arm, one having a width of about 10 to 12 cm and a length of about 10 to 20 cm with a circular opening in the administration portion is exemplified.

The tight-binging band plays a role to tense the skin and give hardness to the skin, and may be made of any material as long as this exerts such an effect. Since the tight-binding band comes into surface contact with the skin, it is preferable to use a material less irritating to the skin, cloth, bandage, woven cloth, rubber and the like are preferably used. More preferably, at least a part of the tight-binding band is made of a stretchable material (stretchable cloth or stretchable rubber).

The hole provided on the tight-binding band forms the micro-needle patch administration portion, and when such tight-binding tool is attached, this gives hardness to the skin located in the hole and forms the micro-needle patch administration site. The hole provided on the tight-binding band may be a hole originally formed on the tight-binding band or a hole formed after attaching the skin tight-binding device. The number of holes is usually one, but may be plural as needed. In a case of a plurality of holes, it is sufficient that the tight-binding band surrounds each hole. A shape of the hole may be appropriately set depending on the size of the micro-needle patch, and is typically circular, but may be elliptical, triangular, square, or polygonal. It is sufficient that the size of the hole is larger than the size of the micro-needle patch.

The hole may give hardness to the skin located in the hole by an action of the surrounding tight-binding band or an action of the fixing tool and the surrounding tight-binding band, but in order to make a pressing force to the skin more efficient, a ring may also be formed around the hole. The ring may have any shape depending on the shape of the hole. A material of the ring may be plastic, a wire, a metal ring, a washer, two or more layers of cloth stitched, rubber and the like. A low-stretchable material is more preferable.

The fixing tool is for fixing the tight-binding band to the skin. As one aspect, the fixing tool may have a belt shape for pressing the tight-binding band from above and bringing the same into close contact with the skin. Alternatively, the fixing tool may have a band shape so as to be integrally connected to the tight-binding band to form the skin tight-binding device. The fixing tool is preferably made of a stretchable material at least partially so that the skin tight-binding device may be fixed according to a shape of the skin to which this is attached, and examples thereof include stretchable cloth, rubber, leather, woven cloth and the like. The tight-binding band and the fixing tool may be made of a stretchable material at least partially.

In order to obtain the skin tight-binding device in which the tight-binding band and the fixing tool are integrally connected to each other, a combination of a less-stretchable (non-stretchable) cloth and a stretchable cloth, a combination of a less-stretchable (non-stretchable) cloth and a stretchable woven cloth, a combination of less-stretchable (non-stretchable) rubber and a rubber band and the like are preferable. It is preferable that an attaching/removing tool such as a hook-and-loop fastener, a hook, and a button is attached to the fixing tool for convenience of attachment and removal. The hook-and-loop fastener also serves to adjust a degree of tension of the skin tight-binding device. As an embodiment of a band-shaped fixing tool integrally connected to the tight-binding band to form the skin tight-binding device, there is a skin tight-binding device in which both ends of the fixing tool have fork shapes (two teeth, three teeth and the like), and corresponding fork-shaped tooth portions are overlapped using a hook-and-loop fastener, a hook, a button and the like to be attached to a limb and the like. In this case, a hole surrounded by fork-shaped teeth is provided.

The size of the fixing tool may be appropriately set depending on a site to be used. In a case of attaching the same to the limb, a length capable of winding around the limb once is sufficient; for example, 20 to 30 cm for the arm. When the tight-binding band and the fixing tool are integrally connected to each other, a total length of the tight-binding band and the fixing tool capable of winding around the limb once is sufficient.

The skin tight-binding device of the present invention positions on the skin such that the tight-binding band surrounds the administration site of the micro-needle patch, and finely adjusts such that the skin on the administration site of the micro-needle patch is exposed in the hole provided on the tight-binding band or in the hole formed after the skin tight-binding device is attached. In this state, this may be fixed to the skin with the fixing tool. After the attachment, it is confirmed that hardness is given to the skin in the hole, and the micro-needle patch is administered. The micro-needle patch may be administered using a device such as a micro-needle applicator, or may be administered by pressing the same with a finger.

The hardness (tension) of the skin may be confirmed in general by visually checking swelling of the skin in the hole. When the skin tight-binding device of the present invention is attached to the skin, even the skin without hardness (tension) may be kept in a tension state to maintain constant hardness (tension) during the attachment.

A degree of skin tension before and after the attachment may be expressed as a "skin deformation ratio". The skin deformation ratio is indicated as a ratio of a degree of skin deformation after the attachment (in millimeter) to a degree of skin deformation before the attachment (in millimeter) (the degree of skin deformation after the attachment/the degree of skin deformation before the attachment). The degree of skin deformation may be measured using a Cutometer (registered trademark, Courage+Khazaka electronic GmbH) most frequently used as a device that measures skin viscoelasticity. Measurement conditions are described in examples.

In the present invention, it is preferable to attach the skin tight-binding device so that the skin deformation ratio is 0.9 or smaller. The skin tight-binding device of the present invention is preferably used such that the skin deformation ratio is 0.9 or smaller. The skin deformation ratio may be adjusted by adjusting a fixing force to the skin by the fixing tool, the material of the tight-binding band, the size of the hole and the like. The skin deformation ratio may be appropriately adjusted also depending on original hardness of the skin of a subject. For the subject with skin tension, a numerical value of the skin deformation ratio may be made relatively larger. Generally, when the skin deformation ratio is in a range from 0.9 to 0.4, preferably from 0.8 to 0.5, the micro-needle array may be certainly inserted into the skin.

The degree of skin tension before and after the attachment may also be expressed as a "depression degree ratio". The depression degree ratio is indicated as a ratio of a degree of skin depression after the attachment (in millimeter) to a degree of skin depression before the attachment (in millimeter) (the degree of skin depression after the attachment/the degree of skin depression before the attachment). In a case where the skin tight-binding device is attached to the upper arm, the degree of skin depression may be expressed by statically placing a cylindrical metal rod so as to be perpendicular to skin on an outer side of the upper arm, the upper arm with the outer side facing upward, and measuring a depth of the skin depressed by a weight of the metal rod. Measurement conditions are described in examples.

In the present invention, it is preferable to attach the skin tight-binding device such that the depression degree ratio is 0.9 or smaller. The skin tight-binding device of the present invention is preferably used such that the depression degree ratio is 0.9 or smaller. The depression degree ratio may be adjusted by adjusting the fixing force to the skin by the fixing tool, the material of the tight-binding band, the size of the hole and the like. The depression degree ratio may be appropriately adjusted also depending on original hardness of the skin of the subject. For the subject with skin tension, a numerical value of the depression degree ratio may be made relatively larger. Generally, when the depression degree ratio is in a range from 0.9 to 0.4, preferably from 0.8 to 0.5, the micro-needle array may be certainly inserted into the skin.

The skin tight-binding device of the present invention may be continuously used while the micro-needle patch is attached to the skin. The skin tight-binding device may be removed from the subject after the administration of the micro-needle patch is finished, or may be removed after inserting the micro-needle array into the skin and then appropriately adhering the micro-needle array to the skin with a protective adhesive tape and the like.

### EXAMPLES

Hereinafter, the present invention is described with illustrative examples; however, the present invention is not limited to the examples.

### (Manufacturing Example 1: Micro-needle Patch)

A micro-needle patch used in this example was produced according to the method disclosed in Example 1 of JP 5852280 B2. The obtained micro-needle patch had a needle length of 400 µm and 1,060 needles in a patch diameter of 10 mm. The micro-needle patch was provided for skin administration in a state of being lined with a protective adhesive tape having a diameter of 2.8 cm. A fluorescent dye and 0.3 µg of resveratrol (purchased from Wako Pure Chemical Industries, Ltd.) were applied to a tip end of 100 µm of the needle together with hyaluronic acid and a glucose base and dried.

### (Administrating Method of Micro-needle Patch)

The micro-needle patch prepared in Manufacturing Example 1 was administered to an outer side of a human upper arm of a volunteer using the spring-type applicator disclosed in Example 1 of JP 6091818 B2. An outer side of an upper right arm of the volunteer was an administration site in following Examples 1-4, and an outer side of an upper left arm of the volunteer was an administration site in following Comparative Examples 1-4. The micro-needle patch was retrieved two hours after the administration.

### (Examples 1-4)

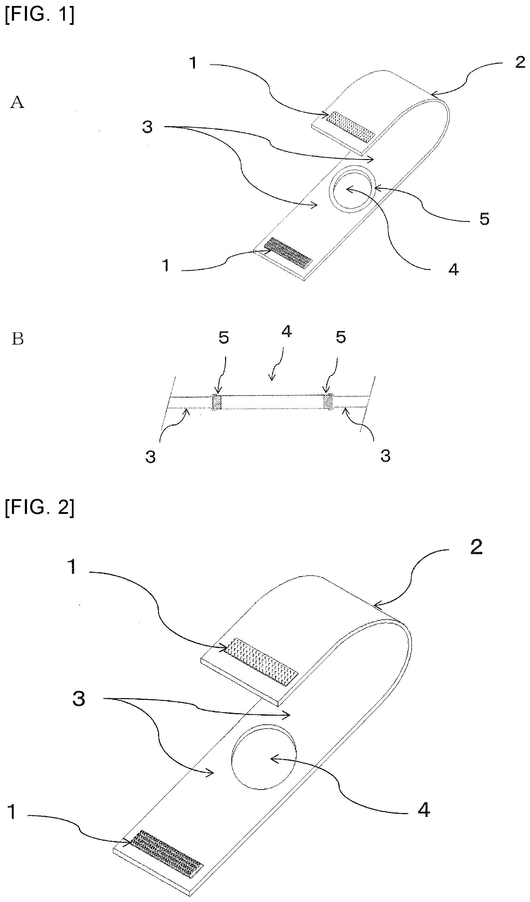

A skin tight-binding device (tight-binding belt) with a hole (diameter of 5 cm) in the center as illustrated in FIGS. 1 and 2 was tightly wound around the upper right arm of the volunteer. Thereafter, the micro-needle patch prepared in Manufacturing Example 1 was administered to skin on the outer side of the upper arm surrounded by the hole in the center using the above-described spring-type applicator.

The tight-binding belt used in Examples 1-2 was a rubber band with a metal wire put around an opening (FIGS. 1A and 1B). The tight-binding belt used in Examples 3-4 was a combination of a low-stretchable cloth and a stretchable woven cloth, and two layers of woven cloth were sewn around the opening, but no wire was used (FIG. 2).

### (Comparative Examples 1-4)

The micro-needle patch prepared in Manufacturing Example 1 was administered to skin on the outer side of the upper left arm of the volunteer participated to Examples 1-4 using the above-described spring-type applicator.

### (Measurement of Degree of Skin Tension)

A most globally popular skin viscoelasticity measuring device (Cutometer (registered trademark) dual MPA580) was used for measuring a degree of skin tension.

A principle of this device is to apply a negative pressure to skin and quantitatively evaluate deformation of the skin due to the pressure. The degree of skin tension may be evaluated by small deformation when the skin is firm and tense.

The degree of skin tension of each volunteer was measured on the skin surrounded by the hole in the center of the belt while attaching the tight-binding belt and on the same site of the skin before attaching the tight-binding belt.

### 1) Skin Hardness Measurement-1

A probe of the Cutometer (registered trademark) was placed at a right angle to the skin in a measurement site and fixed to the skin using a double-sided adhesive seal. Measurement conditions of the degree of skin tension were as follows. They are standard measurement conditions for this device.

Pressure: 450 mbar
Probe opening: diameter of 2 mm
On time: 3 seconds
Off time: 3 seconds

Hardness of the skin is indicated as a degree of skin deformation (mm) when a negative pressure is applied to the skin under the above conditions in this device. Results are illustrated in Table 1.

### 2) Skin Hardness Measurement-2

As another method, the following measurement was performed. After placing the outer side of the upper arm upward, a cylindrical metal rod (weight: 20 g) having a diameter of 2.0 cm was statically placed so as to be perpendicular to the skin of the upper arm. The skin was depressed by the weight of the metal rod, and a depressed depth was measured. With the depression degree becoming smaller, the degree of skin tension was evaluated. Results are illustrated in Table 1.

### (Quantitative Method of Residual Resveratrol)

The micro-needle patches retrieved in Examples 1-4 and Comparative Examples 1-4 were immersed in ethanol, and an amount of residual resveratrol was quantitated by HPLC to examine an insertion degree of the micro-needles into the skin. As a control, an amount of resveratrol extracted from an unused micro-needle patch was similarly quantitated, and this is illustrated in Table 1 as a resveratrol residual rate (%).

Details of the quantitative method were as follows.

An extracting method of resveratrol from the micro-needle patch was performed as follows.
1. The micro-needle patch was placed in a 24-well micro-plate with needles facing upward.
2. To each well, 1 mL of 50% aqueous ethanol solution was added.
3. Resveratrol was extracted after stirring with a plate shaker for about 1 hour.

Resveratrol was quantitated by HPLC as follows.
Resveratrol HPLC Analysis Conditions
Column: Shiseido C18 MG 4.6 mm ID × 250 mm
Eluent: A 0.2% formic acid - water
B 0.2% formic acid - acetonitrile
A : B = 72 : 28
Flow rate: 0.7 mL/min
Column temperature: 60°C
Injection volume: 50 µL
Detector: Fluorescence, excitation wavelength 300 nm, emission wavelength 386 nm

### (Results)

Results of the needle insertion into the skin with and without the tight-binding belt on four volunteers were compared. As a result of microscopic observation of needles, no needle-like residue was observed in all cases with tension. On the contrary, without tension, a residue was observed in a part of the needles, suggesting that the needle insertion into the skin was not complete. A residual amount of loaded resveratrol was quantitated for more accurate comparison.

**[Table 1]**

| Example, Comparative Example | Tight-binding device | Skin hardness evaluation | | Skin deformation ratio with or without tension (*) | Depression degree with or without tension (**) | Resveratrol residual rate (%) |
|---|---|---|---|---|---|---|
| | | Measurement 1 | Measurement 2 | | | |
| | | Skin deformation degree (mm) | Depression degree (mm) | | | |
| Example 1 (Volunteer A) | With wire | 0.12 | 2 | 0.63 | 0.4 | 11 |
| Example 2 (Volunteer B) | With wire | 0.25 | 3 | 0.63 | 0.5 | 8 |
| Example 3 (Volunteer C) | Without wire | 0.18 | 2 | 0.75 | 0.4 | 9 |
| Example 4 (Volunteer D) | Without wire | 0.14 | 3 | 0.78 | 0.6 | 13 |
| Comparative example 1 (Volunteer A) | Not used | 0.19 | 5 | - | - | 33 |
| Comparative example 2 (Volunteer B) | Not used | 0.40 | 6 | - | - | 40 |
| Comparative example 3 (Volunteer C) | Not used | 0.24 | 5 | - | - | 37 |
| Comparative example 4 (Volunteer D) | Not used | 0.18 | 5 | - | - | 45 |

| | | | | | | |
|---|---|---|---|---|---|---|
| (*) For example, calculate by 0.12/0.19 with volunteer A (**) For example, calculate by 2/5 with volunteer A | | | | | | |

### DESCRIPTION OF REFERENCE SYMBOLS

- 1: Hook-and-loop fastener
- 2: Fixing tool
- 3: Tight-binding band
- 4: Hole
- 5: Metal wire ring

## Claims

1. A skin tight-binding device comprising:
a tight-binding band; and
a hole provided on the tight-binding band,
wherein, when the tight-binding band is fixed to skin, the hole forms a micro-needle patch administration portion.

2. The skin tight-binding device according to claim 1, further comprising a fixing tool that fixes the tight-binding band to the skin.

3. The skin tight-binding device according to claim 2, wherein the tight-binding band and the fixing tool are integrally connected to each other to form the skin tight-binding device.

4. The skin tight-binding device according to any one of claims 1 to 3, wherein the hole provided on the tight-binding band is formed after the skin tight-binding device is attached to the skin.

5. The skin tight-binding device according to any one of claims 1 to 4, wherein, in a case of being attached to the skin, a degree of tension of the skin in the hole is 0.9 or smaller as a skin deformation ratio as compared to the degree before the attachment.

6. The skin tight-binding device according to any one of claims 1 to 4, wherein, in a case of being attached to the skin, a degree of tension of the skin in the hole is 0.9 or smaller as a depression degree ratio as compared to the degree before the attachment.

7. The skin tight-binding device according to any one of claims 1 to 6, wherein the tight-binding band or the fixing tool is a stretchable cloth at least partially.

8. The skin tight-binding device according to any one of claims 2 to 7, wherein the fixing tool is a stretchable cloth, and the tight-binding band is a non-stretchable cloth.
